# DEMANDE DE BREVET EUROPEEN

(11) **EP 2 177 893 A1**
(43) Date de publication de la demande: **21.04.2010**
(21) Numéro de dépôt: 09370015.1
(22) Date de dépôt: 13.10.2009
(51) Int. Cl.: G01N 3/08

(54) **Cellule triaxiale polyvalente utilisable pour effectuer des essais**

(30) Priorité: 14.10.2008 FR 0805647
(71) Demandeur: Universite Des Sciences Et Technologies De Lille, 59655 Villeneuve d'Ascq (FR)
(72) Inventeur: Secq, Jean, 59655 Villeneuve d'Ascq (FR)
(74) Mandataire: Duthoit, Michel Georges André

(57) **Abrégé**

La présente invention concerne une cellule triaxiale polyvalente, utilisable pour effectuer des essais sur des matériaux, tes que des géomatériaux, des échantillons de roches, de sols ou de matériaux divers, sur lesquels des mesures physiques doivent être réalisées.

La dite cellule comprenant une structure support (2-5) définissant une chambre (19) d'essai apte à contenir l'échantillon (1) à évaluer, ainsi qu'un ensemble (6-12) de mise en pression traversant le chapeau supérieur (4) de ladite cellule triaxiale.

Selon l'invention, ledit ensemble (6-12) de mise en pression de l'échantillon est composé d'un piston (11) mobile et d'un insert (12) fixe lui servant de guide.

## Description

L'invention concerne une cellule triaxiale polyvalente utilisable pour effectuer des essais sur des géomatériaux, des échantillons de roches, de sols ou de matériaux divers, sur lesquels des mesures physiques doivent être réalisées.

Les échantillons à évaluer peuvent notamment être placés en pression dans une machine d'essai située en laboratoire ou amenée sur un chantier afin de déterminer différents paramètres des sols et roches, ces essais sont par exemple intéressants dans le cas de forages.

La cellule triaxiale objet de l'invention fonctionne comme une presse permettant de placer sous contrainte un échantillon de forme cylindrique disposé dans une cellule. L'appareil de test comporte divers capteurs utiles pour déterminer les réponses de l'échantillon à des sollicitations diverses de pression et de température, par exemple.

Une telle cellule triaxiale est décrite en détail dans le document FR 2 663 121, ce document récapitule l'art antérieur connu en la matière.

Une cellule triaxiale telle que décrite dans le document mentionné précédemment comporte un piston de surface déterminée sur laquelle est appliquée une pression pouvant être variable et contrôlée, un tel dispositif est essentiellement adapté pour des analyses sur des échantillons de grandes dimensions.

Actuellement, l'évolution des techniques d'essais et les difficultés d'obtention des carottes de grands diamètres amènent les expérimentateurs à devoir utiliser des échantillons de diamètres variables et fréquemment des échantillons de petites tailles pour lesquels le piston décrit dans le document FR 2 663 121 est assez mal adapté.

L'invention a donc pour objectif de résoudre cette difficulté principale en proposant un dispositif de mise en pression de l'échantillon qui se substitue au piston existant.

La présente invention concerne une cellule triaxiale polyvalente, utilisable pour effectuer des essais sur des matériaux, comprenant une structure support définissant une chambre d'essai apte à contenir l'échantillon à évaluer, ainsi qu'un ensemble de mise en pression traversant le chapeau supérieur de ladite cellule triaxiale, **caractérisée en ce que** l'ensemble de mise en pression de l'échantillon est composé d'un piston mobile et d'un insert fixe lui servant de guide.

Les avantages de la cellule triaxiale selon l'invention sont multiples :
- des échantillons de petites tailles peuvent être soumis aux tests dans la cellule, avec une pression adaptée, ce qui n'est pas réalisable avec le matériel existant,
- le dispositif proposé permet de substituer rapidement le piston existant avec un dispositif selon l'invention comportant un insert, et donc réaliser avec le même ensemble de test des essais sur des matériaux de différentes dimensions en conservant les avantages spécifiques de ce type de cellule indiqués dans le document FR 2 663 121, notamment l'autonomie et l'auto-compensation,
- à chaque diamètre différent peut correspondre un insert différent et donc des moyens de pression précisément adaptés aux échantillons à évaluer sans qu'il soit nécessaire de modifier le banc de test de manière coûteuse.

D'autres caractéristiques et avantages de l'invention se dégageront de la description qui va suivre en regard des dessins annexés qui ne sont donnés qu'à titre d'exemples non limitatifs.
La figure 1 est une vue en coupe d'une cellule triaxiale selon l'art antérieur décrit dans le document FR 2 663 121.
La figure 2 illustre en coupe une cellule triaxiale selon l'invention.
La figure 3 montre en détail l'ensemble insert - piston représentatif de l'invention.

La cellule triaxiale selon l'invention est globalement similaire à celle décrite dans le document FR 2 663 121 et s'y réfère pour une description plus détaillée.

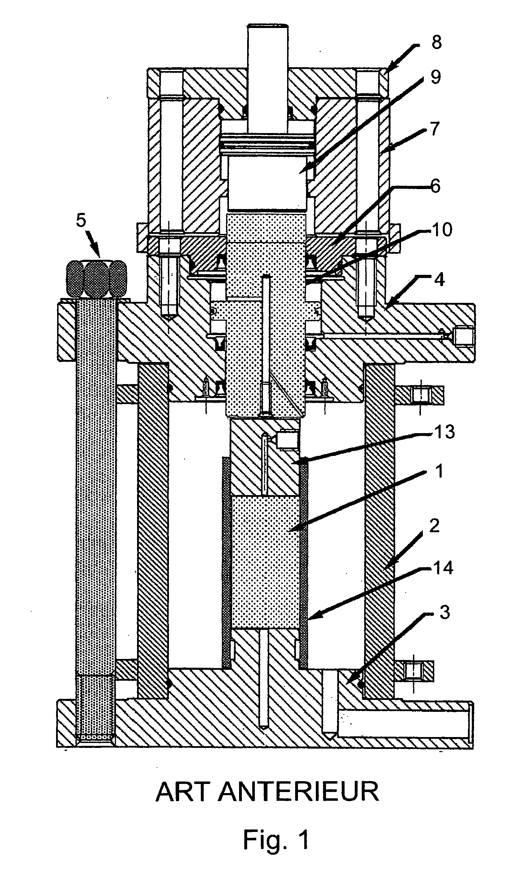

Une telle cellule illustrée sur la figure 1 comporte de nombreux points communs avec la cellule selon l'invention.

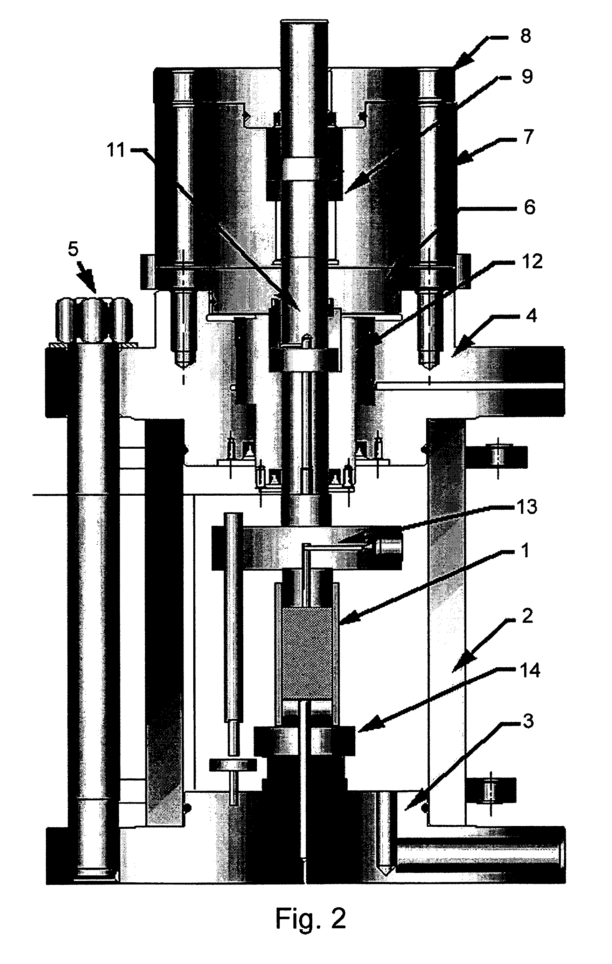

Ladite cellule, qu'elle soit selon l'art antérieur représenté sur la figure 1, ou selon la présente invention représentée sur la figure 2, comprend une structure support 2-5 définissant une chambre d'essai 19 apte à contenir l'échantillon 1 à évaluer.

Plus précisément, l'échantillon 1 est placé au centre de la chambre d'essai 19 de la structure support formée d'un corps de cellule 2, d'une embase inférieure 3 servant de plan de pose, laquelle embase inférieure 3 étant rigidement liée à un chapeau supérieur 4 par l'intermédiaire de tirants 5.

Le corps de cellule 2 présente intérieurement une cavité qui réserve entre sa paroi latérale et celle de l'échantillon 1 ladite chambre d'essai 19, périphérique, reliée à un circuit de mise en pression et de vidange contrôlée de la chambre.

Dans les cellules représentées sur les figures 1 et 2, le chapeau supérieur 4, démontable, sert de support pour un ensemble de mise en pression, maintenu au niveau de l'appareil par un chapeau piston 6, une chambre piston supérieure 7 et un chapeau de chambre supérieure 8.

La contrainte sur l'échantillon 1, dans le but des essais physiques à réaliser, est appliquée axialement par un piston auxiliaire 9 qui transmet la pression soit directement sur le piston 10, dans le cas de l'art antérieur décrit dans le document FR 2 663 121, soit sur un ensemble constitué d'un nouveau piston 11 inséré dans un insert 12 dans le cadre du dispositif selon l'invention.

Entre les pistons 10 ou 11 et l'échantillon 1 est placée une embase drainante 13 comportant un canal permettant l'introduction d'un fluide dans l'échantillon à analyser ou le drainage de liquides inclus.

L'échantillon 1 est placé dans une membrane 14 souple et élastique épousant sa surface latérale, afin de protéger l'échantillon du fluide introduit dans la chambre, ladite membrane 14 peut être composée par exemple d'un manchon obtenu de divers élastomères.

Tel que cela est montré sur les figures 1 et 2, les différences essentielles entre l'art antérieur et la cellule triaxiale selon l'invention se situent au niveau de l'ensemble de mise en pression de l'échantillon.

Dans la figure 1 représentant l'art antérieur, le piston 10 se déplace dans une chambre aménagée dans le chapeau supérieur 4, alors que dans la figure 2, selon l'invention, la course du nouveau piston 11 s'effectue à l'intérieur de l'insert 12, lequel insert est interchangeable et positionné dans un alésage correspondant du chapeau supérieur 4 pour guider, dans un contexte optimal, la course dudit nouveau piston 11.

La cellule triaxiale selon l'invention est remarquable en ce que l'ensemble 6-12 de mise en pression de l'échantillon 1 est, en autre, composé d'un piston 11 mobile et d'un insert 12 fixe, lequel insert 12 servant de guide audit piston 11.

Pour son maintien dans ladite cellule triaxiale, et afin d'être fermement maintenu dans la structure support de l'appareil de test, l'insert 12 est de forme externe tubulaire avec une partie centrale de plus grand diamètre.

Avantageusement, l'insert 12 débouche par sa partie basse 12.1 dans le corps de cellule 2, et par sa partie haute 12.2 vers le chapeau piston 6, et s'emboîte dans le chapeau supérieur 4, sous le chapeau piston 6, tel que cela est représenté sur la figure 2.

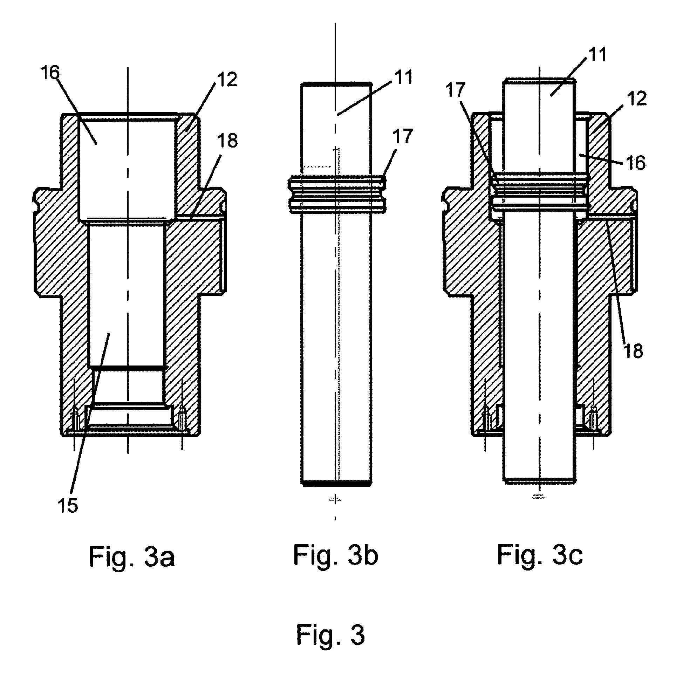

La figure 3 présente en détail l'ensemble insert 12 - piston 11 selon l'invention. Plus précisément, la figure 3a illustre l'insert 12, la figure 3b montre le piston 11, l'ensemble dans sa configuration de fonctionnement étant représenté sur la figure 3c.

Selon l'invention, et en référence à la figure 3a, l'insert 12 est formé, dans sa partie intérieure, par deux chambres cylindriques 15 et 16, de diamètres différents, placées l'une au-dessus de l'autre, et prévues pour permettre le passage du piston 11, ainsi que son maintien en coulissement.

Tel que cela est visible sur la figure 3c, la chambre inférieure 15 de l'insert 12 est de diamètre ajusté, juste supérieur à celui du piston 11, pour permettre son introduction et son déplacement.

Selon l'invention, la chambre supérieure 16 de l'insert 12 est de diamètre plus important que la chambre inférieure 15.

Afin d'assurer une bonne étanchéité le piston 11 comporte un joint torique 17 pour son déplacement dans la chambre supérieure 16. Le montage forme également une butée pour l'avancée du piston en direction de l'échantillon 1 dans la partie basse de ladite chambre supérieure 16.

L'étanchéité étant nécessaire en raison de la présence d'un fluide lubrifiant à au niveau de la chambre inférieure 15 et de la partie de la chambre supérieure située sous le joint torique 17.

Selon l'invention, la partie basse de la chambre supérieure 16, sous le joint 17, est connectée à un canal 18 permettant l'injection d'un fluide actionnant la remontée du piston 11 ou jouant le rôle d'un évent pour purger l'air.

Ainsi configuré, l'ensemble de mise en pression composé du piston 11 et de l'insert 12 est apte à remplacer le piston traditionnellement installé, notamment selon le document FR 2.663.121 précité, sur ce type d'appareil de test, et permettre, en raison du diamètre réduit du piston 11, des essais avec des carottes échantillonnées de dimensions réduites.

Avantageusement, au sens de l'invention, au niveau de l'ensemble de mise en pression 6-9, le piston 11 et l'insert 12 sont amovibles de manière à être remplacés en cas de besoin soit par un piston unique, permettant de tester des échantillons plus grands, soit par un autre ensemble piston - insert comportant un diamètre de piston différent, adapté précisément à la taille de l'échantillon.

Plusieurs variantes de la cellule triaxiale sont possibles, il peut notamment être envisagé, comme cela a déjà été décrit, des diamètres de piston plus ou moins importants pour un diamètre constant de l'insert, sans sortir du cadre de l'invention.

Avantageusement la forme externe de l'insert 12 est prévue pour s'emboîter et s'ajuster dans les organes adjacents, il peut toutefois être prévu un forme externe ou une structure d'emboîtement différente de celle décrite sans sortir du cadre de l'invention.

Les moyens d'étanchéité du piston dans la chambre supérieure, le diamètre de cette chambre supérieure et les moyens pour la remontée du piston peuvent être choisis de diverses manières sans sortir du cadre de l'invention.

Il est envisageable d'installer le dispositif de mise en pression sur différentes presses et divers appareillages d'essais fonctionnant de manière équivalente à la cellule triaxiale sans sortir de l'invention.

Bien entendu, l'invention n'est pas limitée aux modes de réalisation décrits et représentés à titre d'exemples, mais elle comprend aussi tous les équivalents techniques ainsi que leurs combinaisons.

## Revendications

1. Cellule triaxiale polyvalente, utilisable pour effectuer des essais sur des matériaux, comprenant une structure support (2-5) définissant une chambre (19) d'essai apte à contenir l'échantillon (1) à évaluer, ainsi qu'un ensemble (6-12) de mise en pression traversant le chapeau supérieur (4) de ladite cellule triaxiale, **caractérisée en ce que** ledit ensemble (6-12) de mise en pression de l'échantillon est composé d'un piston (11) mobile et d'un insert (12) fixe lui servant de guide.

2. Cellule triaxiale selon la revendication 1, dans laquelle l'insert (12) est de forme externe tubulaire avec une partie centrale de plus grand diamètre pour son maintien dans ladite cellule triaxiale.

3. Cellule triaxiale selon la revendication 2, dans laquelle l'insert (12) débouche par sa partie basse (12.1) dans le corps de cellule (2), et par sa partie haute (12.2) vers le chapeau piston (6), et s'emboîte dans le chapeau supérieur (4).

4. Cellule triaxiale selon la revendication 3, dans laquelle l'insert (12) est formé, dans sa partie intérieure, par deux chambres cylindriques (15, 16) de diamètres différents placées l'une au-dessus de l'autre, et prévues pour permettre le passage du piston (11), ainsi que son maintien en coulissement.

5. Cellule triaxiale selon la revendication 4, dans laquelle chambre inférieure (15) de l'insert (12) est de diamètre ajusté, juste supérieur à celui du piston (11), pour permettre son introduction et son déplacement.

6. Cellule triaxiale selon la revendication 5, dans laquelle la chambre supérieure (16) de l'insert (12) est de diamètre plus important que la chambre inférieure (15).

7. Cellule triaxiale selon la revendication 6, dans laquelle le piston (11) comporte un joint torique (17) pour son déplacement dans la chambre supérieure (16) de l'insert (12), lequel joint (17) et son montage formant butée pour l'avancée du piston (11) en direction de l'échantillon (1) dans la partie basse de ladite chambre supérieure (16).

8. Cellule triaxiale selon la revendication 7, dans laquelle la partie basse de la chambre supérieure (16) est connectée à un canal (18) permettant l'injection d'un fluide actionnant la remontée du piston (11) ou jouant le rôle d'un évent pour purger l'air.

9. Cellule triaxiale selon l'une quelconque des revendications précédentes, dans laquelle au niveau de l'ensemble de mise en pression (6-9), le piston (11) et l'insert (12) sont amovibles de manière à être remplacés en cas de besoin soit par un piston (10) unique soit par un autre ensemble piston (11) - insert (12) comportant un diamètre de piston (11) différent.
